# NEUE EUROPÄISCHE PATENTSCHRIFT

(11) **EP 2 508 521 B2**
(45) Veröffentlichungstag und Bekanntmachung des Hinweises auf die Entscheidung über den Einspruch: **07.09.2022**
(45) Hinweis auf die Patenterteilung: 30.12.2015
(21) Anmeldenummer: 12155662.5
(22) Anmeldetag: 12.10.2004
(51) Int. Cl.: C07D 405/12, A61K 31/505, A61P 35/00

(54) **Dimaleat einer Aminocrotonylverbindung und Verfahren zu ihrer Herstellung**
Method for the production of amino crotonyl compounds
Procédé pour la production de composés crotonyl aminés

(30) Priorität: 17.10.2003 DE 10349113
(43) Veröffentlichungstag der Anmeldung: 10.10.2012
(62) Teilanmeldung aus: 04765927.1
(73) Patentinhaber: Boehringer Ingelheim International GmbH, 55216 Ingelheim am Rhein (DE); Boehringer Ingelheim Pharma GmbH & Co. KG, 55216 Ingelheim am Rhein (DE)
(72) Erfinder: Rall, Werner, 55216 INGELHEIM AM RHEIN (DE); Kulinna, Christian, 55216 INGELHEIM AM RHEIN (DE); Schnaubelt, Juergen, 55216 INGELHEIM AM RHEIN (DE); Sieger, Peter, 55216 INGELHEIM AM RHEIN (DE); Soyka, Rainer, 55216 INGELHEIM AM RHEIN (DE)
(74) Vertreter: Hoffmann Eitle

(56) Entgegenhaltungen:
- WO-A-00/51991
- WO-A-02/50043

## Beschreibung

Die Erfindung betrifft ein Verfahren zur Herstellung von 4-[(3-Chlor-4-fluorphenyl)amino]-6-{[4-(N,N-dimethylamino)-1-oxo-2-buten-1-yl]amino}-7-((*S*)-tetrahydrofuran-3-yloxy)-chinazolin Dimaleat, sowie 4-[(3-Chlor-4-fluorphenyl)amino]-6-{[4-(N,N-dimethylamino)-1-oxo-2-buten-1-yl]amino}-7-((S)-tetrahydrofuran-3-yloxy)-chinazolin Dimaleat und dessen Verwendung zur Herstellung von Arzneimitteln.

4-[(3-Chlor-4-fluorphenyl)amino]-6-{[4-(N,N-dimethylamino)-1-oxo-2-buten-1-yl]-amino}-7-((*S*)-tetrahydrofuran-3-yloxy)-chinazolin besitzt folgende Struktur: 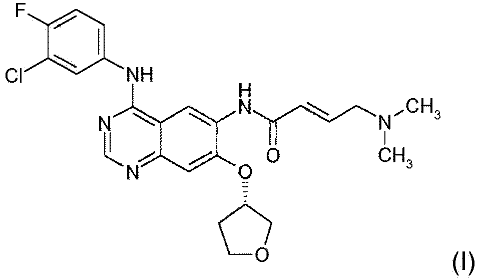 und ist bereits aus der WO 02/50043 bekannt, in der Verbindungen mit wertvollen pharmakologischen Eigenschaften beschrieben werden, zu denen insbesondere eine Hemmwirkung auf die durch Tyrosinkinasen vermittelte Signaltransduktion und eine Hemmwirkung auf die durch den Epidermal Growth Factor-Rezeptor (EGF-R) vermittelte Signaltransduktion gehören. Daher sind Verbindungen diese Typs zur Behandlung von Krankheiten, insbesondere zur Behandlung von Tumorerkrankungen, von Erkrankungen der Lunge und der Atemwege und von Erkrankungen des Magen-Darm-Traktes und der Gallengänge und -blase geeignet.

In der WO 02/50043 wird ein Herstellverfahren offenbart, bei dem Aminocrotonylverbindungen (IV) wie beispielsweise 4-[(3-Chlor-4-fluorphenyl)amino]-6-{[4-(N,N-dimethylamino)-1-oxo-2-buten-1-yl]amino}-7-((*S*)-tetrahydrofuran-3-yloxy)-chinazolin in einer Eintopfreaktion aus dem entsprechende Anilinbaustein (II), Bromcrotonsäure (III), Oxalylchlorid und einem sekundärem Amin hergestellt werden (s. Schema 1).

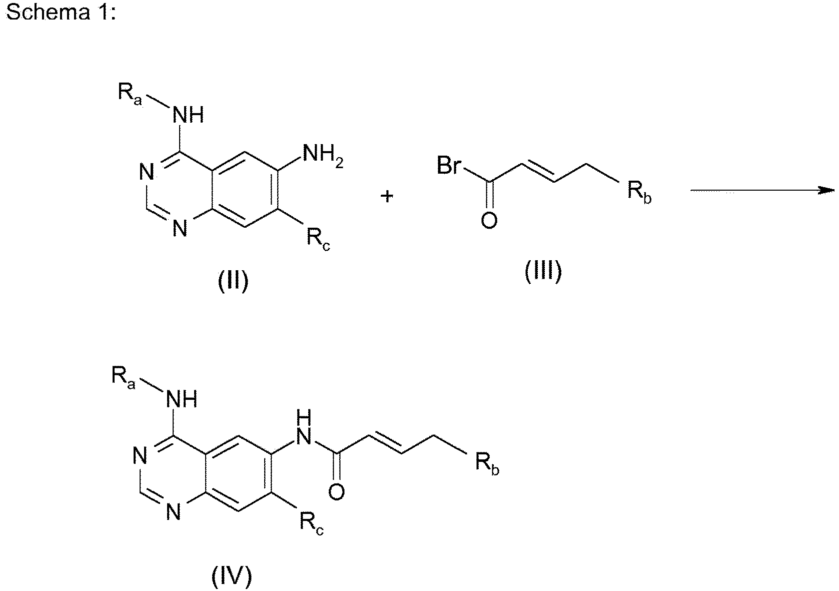

Bei diesem Verfahren lag die Ausbeute bei maximal 50 %. Zudem erfolgte die Reinigung in der Regel mittels Säulenchromatographie. Daher war das Verfahren für die Herstellung von 4-[(3-Chlor-4-fluorphenyl)amino]-6-{[4-(N,N-dimethylamino)-1-oxo-2-buten-1-yl]amino}-7-((*S*)-tetrahydrofuran-3-yloxy)-chinazolin im technischen Maßstab nicht geeignet. Des weiteren hatte die Methode den Nachteil, dass Bromcrotonsäure in größeren Mengen nicht kommerziell erhältlich ist und auch der entsprechenden Bromcrotonsäuremethylester nur in ca. 80 %-iger Reinheit verfügbar ist. Diese Umstände widersprechen ebenfalls der Eignung dieses Verfahrens für die technische Herstellung von 4-[(3-Chlor-4-fluorphenyl)amino]-6-{[4-(N,N-dimethylamino)-1-oxo-2-buten-1-yl]amino}-7-((*S*)-tetrahydrofuran-3-yloxy)-chinazolin.

Im Lichte der oben beschriebenen Nachteile des bekannten Herstellungsverfahrens ist es die Aufgabe der vorliegenden Erfindung, ein Verfahren bereitzustellen, das die Herstellung von 4-[(3-Chlor-4-fluorphenyl)amino]-6-{[4-(N,N-dimethylamino)-1-oxo-2-buten-1-yl]amino}-7-((*S*)-tetrahydrofuran-3-yloxy)-chinazolin Dimaleat unter Verwendung leicht zugänglicher Ausgangsstoffe in hoher Reinheit und ohne großen technischen Aufwand erlaubt. Dieses neue Verfahren soll also auch für die Synthese im technischen Maßstab und damit für die kommerzielle Anwendung geeignet sein. Diese Aufgabe wird durch das erfindungsgemäße Verfahren gelöst. Neben der technischen Durchführbarkeit in hohen Ausbeuten sind sehr gute chemische Reinheiten und ein niedriger cis-Gehalt von unter 0,1 % weitere Vorteile des erfindungsgemäßen Syntheseweges.

Gemäß dem erfindungsgemäßen Verfahren wird die entsprechende Aminoarylverbindung (V) mit einer Di-(C₁₋₄-alkyl)-phosphonoessigsäure, bevorzugt mit Diethylphosphonoessigsäure, in geeigneten Lösungsmitteln, nach entsprechender Aktivierung bevorzugt mit 1,1-Carbonyldiimidazol, 1,1-Carbonylditriazol oder Propanphosphonsäureanhydrid, besonders bevorzugt mit 1,1-Carbonyldiimidazol, gemäß Schema 2 umgesetzt. Als Lösungsmittel können beispielsweise Tetrahydrofuran (THF), Dimethylformamid (DMF) oder Ethylacetat verwendet werden.

Die Aktivierung kann nach allen gängigen Möglichkeiten zu Amidknüpfung erfolgen, also beispielsweise mit 1,1-Carbonyldiimidazol, 1,1-Carbonylditriazol, DCC (N,N-Dicyclohexylcarbodiimid), EDC (N'-(Dimethylaminopropyl)-N-ethylcarbodiimid), TBTU O-(Benzotriazol-1-yl)-N,N,N',N'-tetramethyluroniumtetrafluroborat, Thiazolidin-2-thione oder durch Überführung in das entsprechende Säurechlorid, etwa mit Hilfe von Thionylchlorid. Gegebenenfalls wird die Aktivierung unter Verwendung von organischen Basen wie Triethylamin oder Pyridin durchgeführt, wobei zusätzlich DMAP (Dimethylaminopyridin) zugesetzt werden kann. Als Lösungsmitteln kommen DMF, THF, Essigester, Toluol, chlorierte Kohlenwasserstoffe oder deren Gemisch in Frage.

In den nachstehenden Formeln bedeuten
X ein Stickstoffatom,
Rₐ eine 3-Chlor-4-fluorphenylgruppe und
R¹ eine Ethylgruppe.

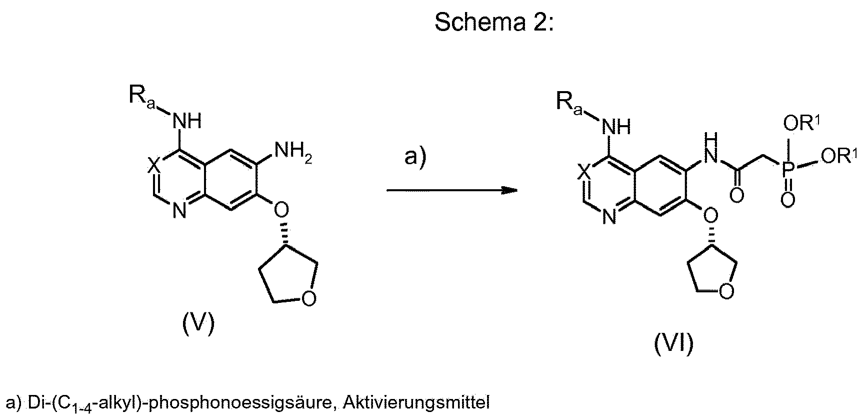

Das so in hoher Ausbeute und hoher Reinheit erhaltene Arylamid (VI) wird mit dem entsprechenden 2-Aminoacetaldehyd unter Einsatz geeigneter organischer oder anorganischer Basen im Sinne einer Wittig-Horner-Emmons-Reaktion umgesetzt (Schema 3). Diese Umsetzung kann direkt oder nach Isolierung der Verbindung (VI), beispielsweise durch Ausfällen mittels Zugabe von beispielsweise *tert*-Butylmethylether, erfolgen. Zu den geeigneten Basen gehören beispielsweise DBU (1,5-Diaza-bicyclo[4.3.0]non-5-en), Natriumhydroxid und Kaliumhydroxid, bevorzugt sind Natriumhydroxid und Kaliumhydroxid, besonders bevorzugt ist Kaliumhydroxid. Anstelle des Aldehyds kann auch ein entsprechendes Äquivalent, z.B. ein Hydrat oder Acetal, aus dem der Aldehyd freigesetzt wird (vorab oder *in situ*), verwendet werden.

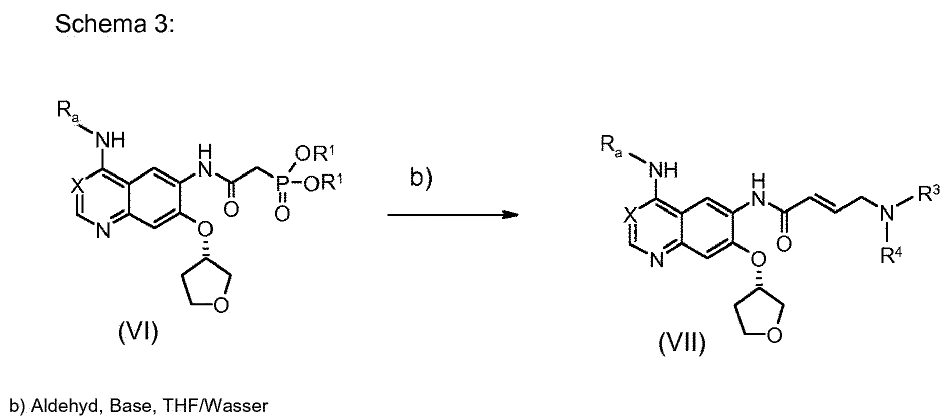

Als Acetale können beispielsweise Verbindungen des folgenden allgemeinen Typs eingesetzt werden: 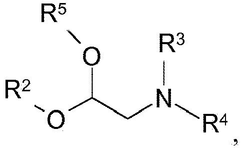 in denen
R³ und R⁴ jeweils eine Methylgruppe und
R² und R⁵ jeweils eine Ethylgruppe bedeuten.

Das so erhaltene 4-[(3-Chlor-4-fluorphenyl)amino]-6-{[4-(N,N-dimethylamino)-1-oxo-2-buten-1-yl]amino}-7-((*S*)-tetrahydrofuran-3-yloxy)-chinazolin der Formel (I) kann anschließend gemäß Schema 4 in sein Dimaleatsalz der Strukturformel (Ia) überführt werden. Dazu wird die Verbindung (I) in einem geeigneten Lösungsmittel, wie beispielsweise Methanol, Isopropanol, n-Butanol oder Ethanol, gegebenenfalls unter Zusatz von Wasser, bevorzugt Ethanol, gelöst und unter Erwärmung mit kristalliner Maleinsäure oder einer Maleinsäurelösung versetzt. Bei Verwendung von Ethanol als Lösungsmittel arbeitet man bevorzugt bei einer Temperatur von zwischen 60 und 75 °C unter Verwendung einer ethanolischen Maleinsäurelösung. Die Reaktionsbedingungen werden bevorzugt so gewählt, dass das gewünschte Salz möglichst schnell auskristallisiert. Vorzugsweise werden ca. 2 Äquivalente Maleinsäure verwendet. Nach einsetzender Kristallisation wird auf Raumtemperatur abgekühlt, nachgerührt und das Kristallisat bestehend aus der Verbindung (Ia) abgetrennt.

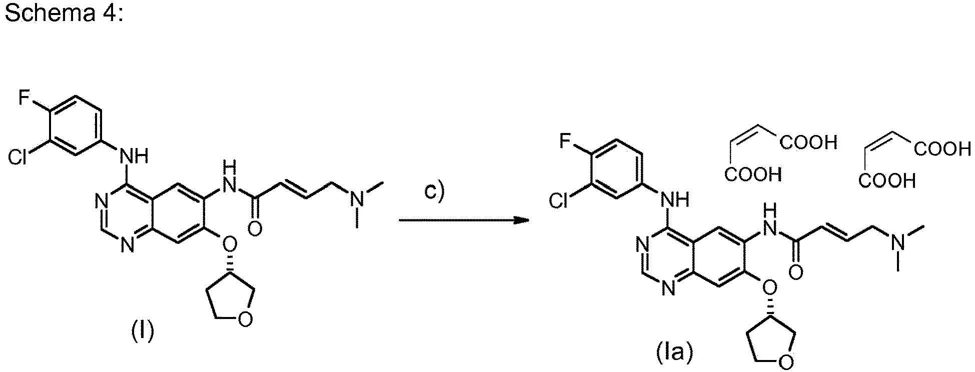

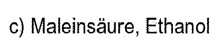

Die Ausgangsverbindung der Formel (V) kann beispielsweise wie folgt nach an sich literaturbekannten Verfahren hergestellt werden:

Zur Herstellung der Verbindung (V) mit X = N geht man wie folgt vor: Ausgehend von kommerziell erhältlicher 4-Chlor-anthranilsäure (VIII; X' = Cl) wird durch Umsetzung mit Formamidin-Acetat das Chinazolinon (IX) erhalten, welches anschließend unter Verwendung von Schwefelsäure und konzentrierter Salpetersäure nitriert wird (Schema 5b). Alternativ kann auch von 4-Fluor-anthranilsäure ausgegangen werden.

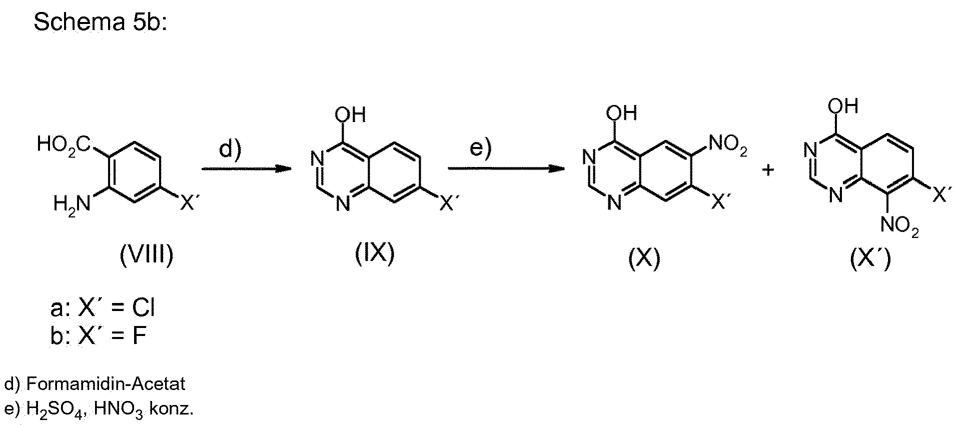

Das gewünschte Regioisomer (X) der so erhaltenen Nitrierungsprodukte wird dann chloriert, und das Chlorierungsprodukt (XI) wird *in situ* mit dem entsprechendem Amin umgesetzt (Schema 6).

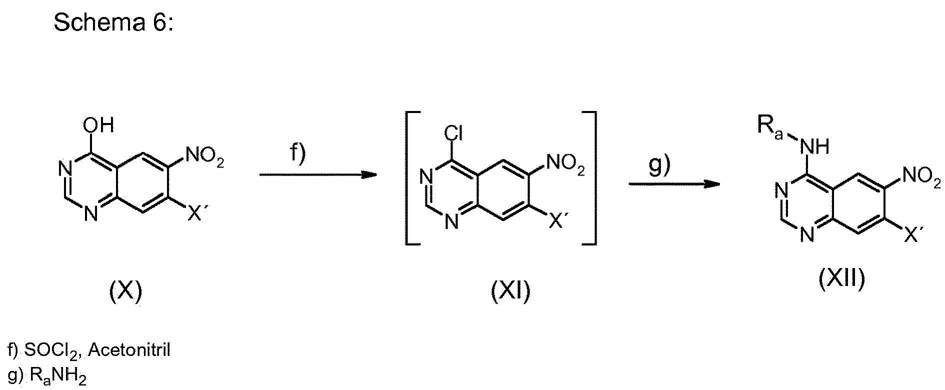

Die so erhaltene Verbindung der Formel (XII) wird mit (S)-(+)-3-Hydroxytetrahydrofuran zu Verbindung (XIII) umgesetzt. Hydrierung der Verbindung (XIII) bzw. die Verbindung (XVIII) aus Schema 5a ergibt dann die Ausgangsverbindung (V) (Schema 7).

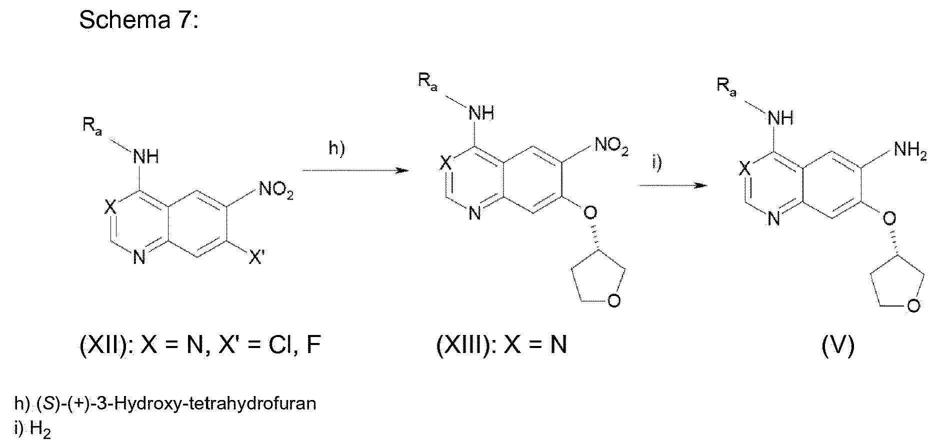

Ein weiterer Erfindungsgegenstand ist das 4-[(3-Chlor-4-fluorphenyl)amino]-6-{[4-(N,N-dimethylamino)-1-oxo-2-buten-1-yl]amino}-7-((*S*)-tetrahydrofuran-3-yloxy)-chinazolin Dimaleat gemäß Anspruch 4. Dieses Salz ist für die pharmazeutische Verwendung besonders geeignet, da es in einer kristallinen Modifikation existiert, die wasserfrei und sehr stabil ist.

Für die pharmazeutische Anwendung muß ein Wirkstoff nicht nur die gewünschte Wirkung zeigen, sondern außerdem noch weiteren Anforderungen gerecht werden, um als Arzneimittel zum Einsatz gelangen zu können. Diese Parameter sind zu einem großen Teil mit der physikochemischen Beschaffenheit des Wirkstoffs verbunden.

Ohne Einschränkung darauf sind Beispiele dieser Parameter die Wirkstabilität des Ausgangsstoffes unter verschiedenen Umgebungsbedingungen, die Stabilität im Verlauf der Herstellung der pharmazeutischen Formulierung sowie die Stabilität in den Endzusammensetzungen des Arzneimittels. Der zur Herstellung der Arzneimittel-zusammensetzungen verwendete Arzneiwirkstoff sollte daher eine hohe Stabilität aufweisen, die auch unter verschiedenen Umgebungsbedingungen gewährleistet sein muß. Dies ist zwingend erforderlich, um zu verhindern, dass Arzneimittelzusammensetzungen Verwendung finden, in denen neben tatsächlichem Wirkstoff beispielsweise Abbauprodukte desselben enthalten sind. In einem solchen Fall könnte ein in pharmazeutischen Formulierungen vorgefundener Gehalt an Wirkstoff niedriger sein als spezifiziert.

Die Absorption von Feuchtigkeit vermindert den Gehalt an Arzneiwirkstoff wegen der durch die Wasseraufnahme verursachten Gewichtszunahme. Zur Aufnahme von Feuchtigkeit neigende Arzneimittel müssen während der Lagerung vor Feuchtigkeit geschützt werden, beispielsweise durch Zusatz von geeigneten Trockenmitteln oder durch Lagerung des Arzneimittels in einer vor Feuchtigkeit geschützten Umgebung. Zudem kann die Aufnahme von Feuchtigkeit den Gehalt an Arzneiwirkstoff während der Herstellung vermindern, wenn das Arzneimittel der Umgebung ohne jeglichen Schutz vor Feuchtigkeit ausgesetzt wird. Vorzugsweise sollte ein Arzneimittelwirkstoff daher nur in geringem Maße hygroskopisch sein.

Da die Kristallmodifikation eines Wirkstoffs für den reproduzierbaren Wirkstoffgehalt einer Darreichungsform von Bedeutung ist, besteht die Notwendigkeit, eventuell existierenden Polymorphismus eines kristallin vorliegenden Wirkstoffs bestmöglich aufzuklären. Sofern verschiedene polymorphe Modifikationen eines Wirkstoffs auftreten, sollte gewährleistet sein, dass sich die kristalline Modifikation der Substanz in der späteren Arzneimittelzubereitung nicht verändert. Andernfalls könnte dies die reproduzierbare Wirksamkeit des Medikaments nachteilig beeinflussen. Bevorzugt sind vor diesem Hintergrund Wirkstoffe, die nur durch geringen Polymorphismus gekennzeichnet sind.

Ein weiteres Kriterium, welches je nach Wahl der Formulierung oder nach Wahl des Herstellungsverfahrens der Formulierung von unter Umständen herausragender Bedeutung ist, ist die Löslichkeit des Wirkstoffs. Werden beispielsweise Arzneimittellösungen (etwa für Infusionen) bereitgestellt, so ist eine ausreichende Löslichkeit des Wirkstoffs in physiologisch verträglichen Lösemitteln unverzichtbar. Auch für oral zu applizierende Arzneimittel ist eine ausreichende Löslichkeit des Wirkstoffs von großer Wichtigkeit.

Der vorliegenden Erfindung liegt die Aufgabe zugrunde, einen Arzneimittelwirkstoff bereitzustellen, der nicht nur durch eine hohe pharmakologische Wirksamkeit gekennzeichnet ist, sondern ferner den vorstehend genannten physikochemischen Anforderungen bestmöglich gerecht wird.

Diese Aufgabe wird durch 4-[(3-Chlor-4-fluorphenyl)amino]-6-{[4-(N,N-dimethylamino)-1-oxo-2-buten-1-yl]amino}-7-((*S*)-tetrahydrofuran-3-yloxy)-chinazolin Dimaleat gemäß Anspruch 4 gelöst.

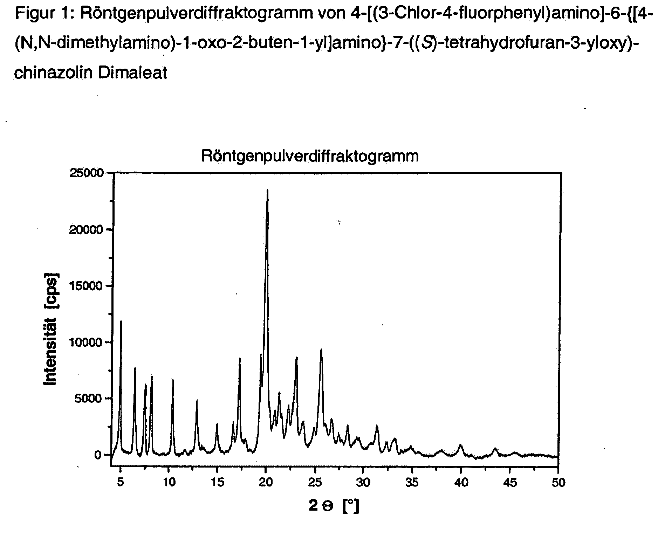

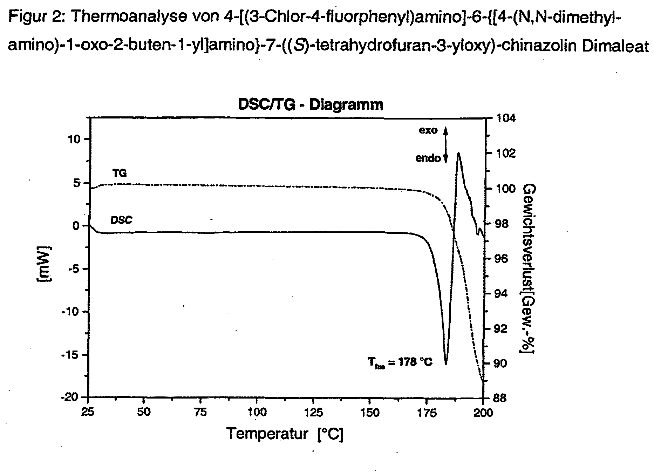

4-[(3-Chlor-4-fluorphenyl)amino]-6-{[4-(N,N-dimethylamino)-1-oxo-2-buten-1-yl]-amino}-7-((*S*)-tetrahydrofuran-3-yloxy)-chinazolin Dimaleat besitzt einen Schmelzpunkt von 178°C (vgl. die in Figur 2 wiedergegebene Thermoanalyse). Das kristalline 4-[(3-Chlor-4-fluorphenyl)amino]-6-{[4-(N,N-dimethylamino)-1-oxo-2-buten-1-yl]-amino}-7-((*S*)-tetrahydrofuran-3-yloxy)-chinazolin Dimaleat wurde mittels Röntgenpulverbeugung näher untersucht. Das erhaltene Diagramm ist in Figur 1 dargestellt. Die nachstehende Tabelle führt die bei dieser Analyse erhaltenen Daten auf:

**Tabelle: Röntgenpulverreflexe und Intensitäten (normiert) des 4-[(3-Chlor-4-fluorphenyl)amino]-6-{[4-(N,N-dimethylamino)-1-oxo-2-buten-1-yl]amino}-7-((S)-tetrahydrofuran-3-yloxy)-chinazolin Dimaleat**

| **2-Θ** [°] | **d-value [Å]** | **intensity I/I₀ [%]** |
|---|---|---|
| 4,91 | 18,0 | 47 |
| 6,42 | 13,8 | 33 |
| 7,47 | 11,8 | 27 |
| 8,13 | 10,9 | 30 |
| 10,37 | 8,53 | 30 |
| 11,69 | 7,56 | 2 |
| 12,91 | 6,85 | 20 |
| 13,46 | 6,58 | 3 |
| 13,66 | 6,48 | 2 |
| 14,94 | 5,93 | 11 |
| 16,58 | 5,34 | 12 |
| 17,19 | 5,15 | 36 |
| 17,87 | 4,96 | 5 |
| 19,43 | 4,57 | 38 |
| 19,91 | 4,46 | 100 |
| 20,84 | 4,26 | 13 |
| 21,33 | 4,16 | 21 |
| 21,58 | 4,12 | 12 |
| 22,25 | 3,992 | 15 |
| 22,94 | 3,873 | 32 |
| 23,67 | 3,756 | 9 |
| 24,82 | 3,584 | 7 |
| 25,56 | 3,482 | 37 |
| 26,71 | 3,335 | 9 |
| 27,46 | 3,245 | 4 |
| 28,37 | 3,143 | 8 |
| 30,71 | 2,909 | 3 |
| 29,31 | 3,045 | 4 |
| 29,57 | 3,019 | 4 |
| 31,32 | 2,854 | 10 |
| 32,31 | 2,769 | 4 |
| 33,10 | 2,705 | 5 |
| 33,90 | 2,643 | 1 |
| 34,84 | 2,573 | 2 |
| 35,71 | 2,512 | 1 |
| 36,38 | 2,467 | 1 |
| 36,96 | 2,430 | 1 |
| 37,99 | 2,367 | 2 |
| 39,94 | 2,255 | 5 |

In der voranstehenden Tabelle steht der Wert "2 Θ [°]" für den Beugungswinkel in Grad und der Wert "dₕₖₗ [A]" für die bestimmten Abstände in A zwischen den Gitterebenen.

Die Röntgenpulverdiagramme wurden im Rahmen der vorliegenden Erfindung mittels eines Bruker D8 Advanced - Diffraktometers, ausgerüstet mit einem PSD-Detektor und einer Cu-Anode als Röntgenquelle (CuK_{α} - Strahlung, λ = 1.5418 A, 40 kV, 40 mA) aufgenommen.

Die folgenden Beispiele sollen die Erfindung näher erläutern:

### Beispiele:

### Beispiel 1

### {[4-(3-Chlor-4-fluor-phenylamino)-7-((S)-tetrahydrofuran-3-yloxy)-chinazolin-6-ylcarbamoyl]-methyl}-phosphonsäure-diethylester

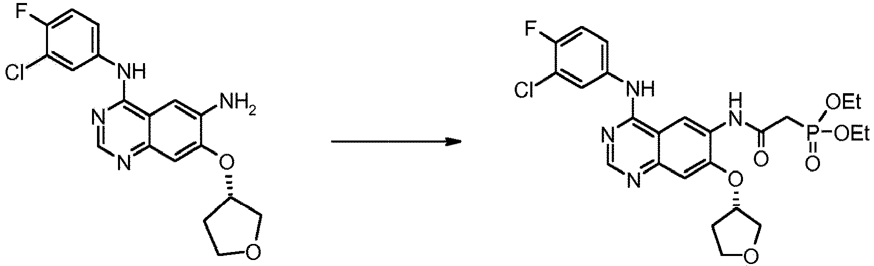

3,58 kg 1,1-Carbonyldiimidazol (22,16 mol) werden in 12,8 Liter Tetrahydrofuran vorgelegt und bei 40 °C mit 4,52 kg (22,16 mol) Diethylphosphonoessigsäure, gelöst in 6,5 Liter Tetrahydrofuran, versetzt. Es wird 30 Minuten lang bei 40 °C nachgerührt. Die so erhaltene Lösung wird als Lösung A bezeichnet.

6,39 kg (17,05 mol) N⁴-(3-Chlor-4-fluor-phenyl)-7-(tetrahydrofuran-3-yloxy)chinazolin-4,6-diamin in 26,5 Liter Tetrahydrofuran vorlegt und bei 40 °C mit der Lösung A versetzt und 2 Stunden bei 30 °C nachgerührt. Zur Suspension werden 64 Liter tert.-Butylmethylether gegeben und nach dem Abkühlen auf 20 °C wird der Niederschlag abzentrifugiert. Es wird mit einer Mischung aus 16 Liter Tetrahydrofuran und 16 Liter tert.-Butylmethylether und anschließend mit 32 Liter Wasser gewaschen und bei 50 °C getrocknet.
Ausbeute: 6,58 kg (69,8%) weiße Kristalle Gehalt: HPLC 99,1 Fl%

### Beispiel 2

### (E)-4-Dimethylamino-but-2-ensäure-[4-(3-chlor-4-fluor-phenylamino)-7-((S)-tetrahydrofuran-3-ylox)-chinazolin-6yl]-amid

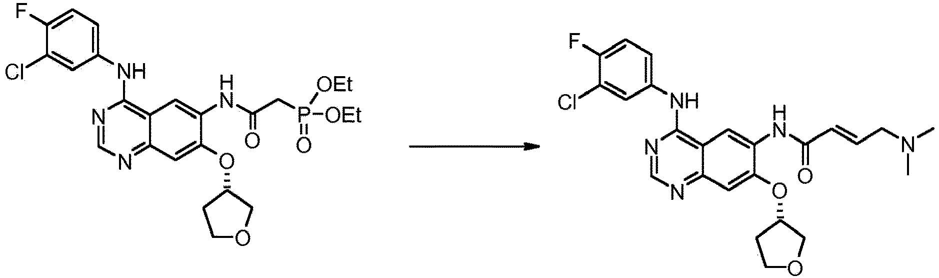

Zu 4,4 Liter Wasser werden 5,6 Liter 30 %-iger Salzsäure (53,17 mol) gegeben. Anschließend werden 4,28 kg 95 %-iges (Dimethylamino)-acetaldehyd-diethylacetal (26,59 mol) bei 30°C innerhalb 20 Minuten zugetropft. Die Reaktionslösung wird 8 Stunden bei 35°C nachgerührt, auf 5 °C abgekühlt und unter Argon aufbewahrt. Diese Lösung wird als Lösung B bezeichnet.

4,55 kg (68,06 mol) Kaliumhydroxid werden in 23,5 Liter Wasser gelöst und auf -5°C abgekühlt. Diese Lösung wird als Lösung C bezeichnet.

5,88 kg (10,63 mol) ((4-(3-Chlor-4-fluor-phenylamino)-7-(tetrahydrofuran-3-yloxy)-chinazolin-6-ylcarbamoyl)-methyl)-phosphonsäure-diethylester und 0,45 kg Lithiumchlorid (10,63 mol) werden in 23,5 Liter Tetrahydrofuran vorgelegt und auf -7 °C abgekühlt. Die kalte Lösung C wird innerhalb 10 Minuten zugegeben. Anschließend wird die Lösung B bei -7 °C innerhalb 1 Stunde zugegeben. Nach einstündigem Nachrühren bei -5 °C wird das Reaktionsgemisch auf 20°C erwärmt und mit 15 Liter Wasser versetzt. Nach dem Abkühlen auf 3 °C wird die Suspension abgesaugt, der Niederschlag mit Wasser gewaschen und getrocknet. Ausbeute: 5,21 kg roh 100 % Wassergehalt: 6,7 %

Die Kristallisation des Rohproduktes erfolgt mit Butylacetat / Methylcyclohexan Ausbeute: 78 % Reinheit HPLC 99,4 FI%, Wassergehalt 5,4 %

### Beispiel 3

### (E)-4-Dimethylamino-but-2-ensäure-(4-(3-chlor-4-fluor-phenylamino)-7-((S)-tetrahydrofuran-3-yloxy)-chinazolin-6yl)-amid Dimaleat

6,0 kg (12,35 mol) (E)-4-Dimethylamino-but-2-ensäure-(4-(3-chlor-4-fluor-phenylamino)-7-((S)-tetrahydrofuran-3-yloxy)-chinazolin-6yl)-amid werden in 84 Liter Ethanol vorgelegt und auf 70 °C erhitzt und mit einer Lösung von 2,94 kg (25,31 mol) Maleinsäure in 36 Liter Ethanol versetzt. Nach einsetzender Kristallisation wird zuerst auf 20 °C abgekühlt und 2 Stunden nachgerührt, dann 3 Stunden bei 0 °C. Der Niederschlag wird abgesaugt, mit 19 Liter Ethanol nachgewaschen und im Vakuum bei 40°C getrocknet.
Ausbeute: 8,11 kg (91,5%)
Smp: 178°C
¹H-NMR (CD₃OD): δ = 2,47 + 2,27 (m+m, 2H), 2,96 (s, 6H), 4,03 (m, 2H), 4,07 + 3,92 (m+m, 2H), 4,18 + 4,03 (m+m, 2H), 5,32 (m, 1H), 6,26 (s, 4H), 6,80 (m, 1H), 6,99 (m, 1 H), 7,27(s, 1 H), 7,30 (t, 1 H), 7,66 (m, 1 H), 7,96 (dd, 1 H), 8,62 (s, 1 H), 9,07 (s, 1 H) ppm

## Patentansprüche

1. Verfahren zur Herstellung des Dimaleats von 4-[(3-Chlor-4-fluorphenyl)amino]-6-{[4-(N,N-dimethylamino)-1-oxo-2-buten-1-yl]aminol-7-((*S*)-tetrahydrofuran-3-yloxy)-chinazolin, umfassend die Schritte a und b
a) Umsetzung einer Verbindung der allgemeinen Formel (V) 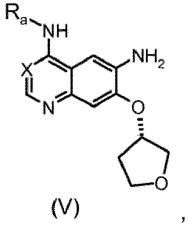
in der X ein Stickstoffatom und
Rₐ die 3-Chlor-4-fluorphenylgruppe bedeuten,
in geeigneten Lösungsmitteln nach entsprechender Aktivierung mit Di-(C1-4-alkyl)-phosphonoessigsäure und
b) Umsetzung der so erhaltenen Verbindung der allgemeinen Formel (VI) 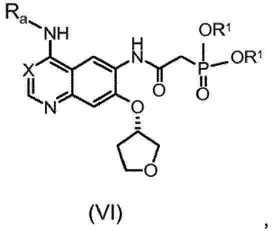
in der X ein Stickstoffatom,
Rₐ die 3-Chlor-4-fluorphenylgruppe und
R¹ eine lineare oder verzweigte C₁₋₄-Alkylgruppe bedeuten,
mit dem Aldehyd der Formel 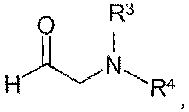
in der R³ und R⁴ jeweils eine Methylgruppe bedeuten,
oder einem entsprechenden Aldehyd-Äquivalent unter Einsatz geeigneter organischer oder anorganischer Basen,
sowie den folgenden Schritt c):
c) Überführung des so erhaltenen 4-[(3-Chlor-4-fluorphenyl)amino]-6-{[4-(N,N-dimethylamino)-1-oxo-2-buten-1-yl]amino}-7-((S)-tetrahydrofuran-3-yloxy)-chinazolins in das Dimaleat durch Umsetzung unter Erwärmung mit Maleinsäure in einem geeigneten Lösungsmittel.

2. Verfahren gemäß Anspruch 1, **dadurch gekennzeichnet, dass** Ethanol oder Isopropanol als Lösungsmittel verwendet werden, gegebenenfalls unter Zusatz von Wasser.

3. Verfahren gemäß Anspruch 1 oder 2, **dadurch gekennzeichnet, dass** mindestens 2 Äquivalente Maleinsäure verwendet werden.

4. Kristallines 4-[(3-Chlor-4-fluorphenyl)amino]-6-{[4-(N,N-dimethylamino)-1-oxo-2-buten-1-yl]-amino}-7-((*S*)-tetrahydrofuran-3-yloxy)-chinazolin Dimaleat, **gekennzeichnet durch** folgende mittels CuK_{α} Strahlung mit einer Wellenlänge von λ = 1,5418 Ä bestimmten Beugungswinkel :
| **2-Θ [°]** | **d-value [Å]** | **intensity I/Iₒ [%]** |
|---|---|---|
| 4.91 | 18.0 | 47 |
| 6.42 | 13.8 | 33 |
| 7.47 | 11.8 | 27 |
| 8.13 | 10.9 | 30 |
| 10.37 | 8.53 | 30 |
| 11.69 | 7.56 | 2 |
| 12.91 | 6.85 | 20 |
| 13.46 | 6.58 | 3 |
| 13.66 | 6.48 | 2 |
| 14.94 | 5.93 | 11 |
| 16.58 | 5.34 | 12 |
| 17.19 | 5.15 | 36 |
| 17.87 | 4.96 | 5 |
| 19.43 | 4.57 | 38 |
| 19.91 | 4.46 | 100 |
| 20.84 | 4.26 | 13 |
| 21.33 | 4.16 | 21 |
| 21.58 | 4.12 | 12 |
| 22.25 | 3.992 | 15 |
| 22.94 | 3.873 | 32 |
| 23.67 | 3.756 | 9 |
| 24.82 | 3.584 | 7 |
| 25.56 | 3.482 | 37 |
| 26.71 | 3.335 | 9 |
| 27.46 | 3.245 | 4 |
| 28.37 | 3.143 | 8 |
| 30.71 | 2.909 | 3 |
| 29.31 | 3.045 | 4 |
| 29.57 | 3.019 | 4 |
| 31.32 | 2.854 | 10 |
| 32.31 | 2.769 | 4 |
| 33.10 | 2.705 | 5 |
| 33.90 | 2.643 | 1 |
| 34.84 | 2.573 | 2 |
| 35.71 | 2.512 | 1 |
| 36.38 | 2.467 | 1 |
| 36.96 | 2.430 | 1 |
| 37.99 | 2.367 | 2 |
| 39.94 | 2.255 | 5 |

5. Arzneimittel enthaltend kristallines 4-[(3-Chlor-4-fluorphenyl)amino]-6-{[4-(N,N-dimethylamino)-1-oxo-2-buten-1-yl]aminol-7-((*S*)-tetrahydrofuran-3-yloxy)-chinazolin Dimaleat nach Anspruch 4 neben gegebenenfalls einem oder mehreren inerten Trägerstoffen und/oder Verdünnungsmitteln.

6. Verwendung von kristallinem 4-[(3-Chlor-4-fluorphenyl)amino]-6-{[4-(N,N-dimethylamino)-1-oxo-2-buten-1-yl]amino}-7-((*S*)-tetrahydrofuran-3-yloxy)-chinazolin Dimaleat nach Anspruch 4 zur Herstellung eines Arzneimittels, das zur Behandlung von benignen oder malignen Tumoren, zu Vorbeugung und Behandlung von Erkrankungen der Atemwege und der Lunge sowie zur Behandlung von Erkrankungen des Magen-Darm-Traktes und der Gallengänge und -blase geeignet ist.

## Claims

1. Method for producing the dimaleate of 4-[(3-chloro-4-fluorophenyl)amino]-6-{[4-(N,N-dimethylamino)-1-oxo-2-butene-1-yl]amino}-7-((S)-tetrahydrofuran-3-yloxy)-quinazoline, comprising the steps a and b
a) reacting a compound of the general formula (V) 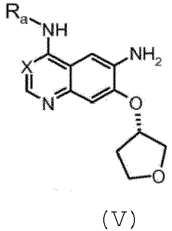
where X is a nitrogen atom and
Rₐ is the 3-chloro-4-fluorophenyl group,
in suitable solvents following appropriate activation with di-(Cl-4-alkyl)-phosphonoacetic acid and
b) reacting the compound of the general formula (VI) thus obtained 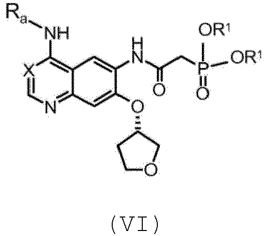
where X is a nitrogen atom,
Rₐ is the 3-chloro-4-fluorophenyl group and
R¹ is a linear or branched C₁₋₄ alkyl group,
with the aldehyde of the formula 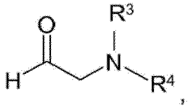
where R³ und R⁴ are in each case a methyl group,
or a corresponding aldehyde equivalent using suitable organic or inorganic bases,
as well as the following step c):
c) converting the 4-[(3-chloro-4-fluorophenyl)amino]-6-{[4-(N,N-dimethylamino)-1-oxo-2-butene-1-yl]amino}-7-((S)-tetrahydrofuran-3-yloxy)-quinazoline thus obtained into the dimaleate by reacting, under heating, with maleic acid in a suitable solvent.

2. Method according to claim 1, **characterised in that** ethanol or isopropanol can be used as solvents, optionally with the addition of water.

3. Method according to claim 1 or 2, **characterised in that** at least 2 equivalents of maleic acid are used.

4. Crystalline 4-[(3-chloro-4-fluorophenyl)amino]-6-{[4-(N,N-dimethylamino)-1-oxo-2-butene-1-yl]-amino}-7-((S)-tetrahydrofuran-3-yloxy)- quinazoline dimaleate, **characterised by** the following diffraction angle determined by means of CuK_{α} radiation with a wavelength of λ = 1.5418 Å:
| **2-Θ [°]** | **d-value [Å]** | **intensity l/lₒ [%]** |
|---|---|---|
| 4.91 | 18.0 | 47 |
| 6.42 | 13.8 | 33 |
| 7.47 | 11.8 | 27 |
| 8.13 | 10.9 | 30 |
| 10.37 | 8.53 | 30 |
| 11.69 | 7.56 | 2 |
| 12.91 | 6.85 | 20 |
| 13.46 | 6.58 | 3 |
| 13.66 | 6.48 | 2 |
| 14.94 | 5.93 | 11 |
| 16.58 | 5.34 | 12 |
| 17.19 | 5.15 | 36 |
| 17.87 | 4.96 | 5 |
| 19.43 | 4.57 | 38 |
| 19.91 | 4.46 | 100 |
| 20.84 | 4.26 | 13 |
| 21.33 | 4.16 | 21 |
| 21.58 | 4.12 | 12 |
| 22.25 | 3.992 | 15 |
| 22.94 | 3.873 | 32 |
| 23.67 | 3.756 | 9 |
| 24.82 | 3.584 | 7 |
| 25.56 | 3.482 | 37 |
| 26.71 | 3.335 | 9 |
| 27.46 | 3.245 | 4 |
| 28.37 | 3.143 | 8 |
| 30.71 | 2.909 | 3 |
| 29.31 | 3.045 | 4 |
| 29.57 | 3.019 | 4 |
| 31.32 | 2.854 | 10 |
| 32.31 | 2.769 | 4 |
| 33.10 | 2.705 | 5 |
| 33.90 | 2.643 | 1 |
| 34.84 | 2.573 | 2 |
| 35.71 | 2.512 | 1 |
| 36.38 | 2.467 | 1 |
| 36.96 | 2.430 | 1 |
| 37.99 | 2.367 | 2 |
| 39.94 | 2.255 | 5 |

5. Medicinal product containing crystalline 4-[(3-chloro-4-fluorophenyl)amino]-6-{[4-(N,N-dimethylamino)-1-oxo-2-butene-1-yl]amino}-7-((S)-tetrahydrofuran-3-yloxy)-quinazoline dimaleate according to claim 4 as well as, optionally, one or more inert carriers and/or diluents.

6. Use of crystalline 4-[(3-chloro-4-fluorophenyl)amino]-6-{[4-(N,N-dimethylamino)-1-oxo-2-butene-1-yl]amino}-7-((S)-tetrahydrofuran-3-yloxy)-quinazoline dimaleate according to claim 4 for the preparation of a medicinal product suitable for the treatment of benign or malignant tumours, for the prevention and treatment of diseases of the respiratory tract and lungs and for the treatment of diseases of the gastrointestinal tract and bile ducts and gall bladder.

## Revendications

1. Procédé servant à la production du dimaléate de 4-[-3-chloro-4-fluorophényl)amino]-6-{[4-(N,N-diméthyl-amino)-1-oxo-2-butène-1-yl]amino}-7-((*S*)-tetrahydro-furan-3-yloxy)-quinazoline, comprenant les étapes a et b suivantes :
a) la réaction d'un composé de la formule générale (V) 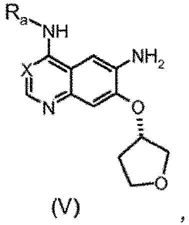 dans laquelle X représente un atome d'azote et Rₐ représente le groupe 3-chloro-4-fluorophényle dans des solvants appropriés selon l'activation correspondante avec l'acide phosphonoacétique de di-(alkyle en C1-4) et
b) la réaction du composé ainsi obtenu de la formule générale (VI) 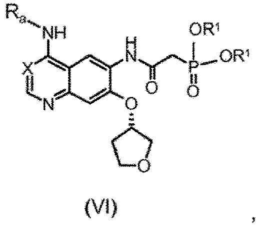
dans laquelle X représente un atome d'azote,
Rₐ le groupe 3-chloro-4-fluorophényle et
R¹ un groupe alkyle en C₁₋₄ linéaire ou ramifié,
avec l'aldéhyde de la formule 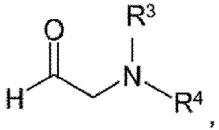
dans laquelle R³ et R⁴ représentent respectivement un groupe méthyle,
ou avec un équivalent correspondent de l'aldéhyde en utilisant des bases organiques ou inorganiques appropriées,
ainsi que l'étape c) qui suit :
c) le transfert de la 4-[(3-chloro-4-fluorophényl)amino]-6-{[4-(N,N-diéthylamino)-1-oxo-2-butène-1-yl]amino}-7-((S)-tétrahydrofuran-3-yloxy)-quinazoline ainsi obtenue dans le dimaléate par réaction en réchauffant avec de l'acide maléique dans un solvant approprié.

2. Procédé selon la revendication 1, **caractérisé en ce que** sont utilisés, en tant que solvants, de l'éthanol ou de l'isopropanol, éventuellement en ajoutant de l'eau.

3. Procédé selon la revendication 1 ou 2, **caractérisé en ce que** sont utilisés au moins 2 équivalents de l'acide maléique.

4. Dimaléate cristallin de 4-[(3-chloro-4-fluorophényl)amino]-6-{[4-(N,N-diméthylamino)-1-oxo-2-butène-1-yl]-aminol-7-((*S*)-tétrahydrofuran-3-yloxy)-quinazoline, **caractérisé par** les angles de diffraction suivants, déterminés au moyen d'un rayonnement CuK_{α} présentant une longueur d'onde de λ = 1,5418 Å :
| **2-Θ [°]** | **valeur d [Å]** | **intensité l/lₒ [%]** |
|---|---|---|
| 4,91 | 18,0 | 47 |
| 6,42 | 13,8 | 33 |
| 7,47 | 11,8 | 27 |
| 8,13 | 10,9 | 30 |
| 10,37 | 8,53 | 30 |
| 11,69 | 7,56 | 2 |
| 12,91 | 6,85 | 20 |
| 13,46 | 6,58 | 3 |
| 13,66 | 6,48 | 2 |
| 14,94 | 5,93 | 11 |
| 16,58 | 5,34 | 12 |
| 17,19 | 5,15 | 36 |
| 17,87 | 4,96 | 5 |
| 19,43 | 4,57 | 38 |
| 19,91 | 4,46 | 100 |
| 20,84 | 4,26 | 13 |
| 21,33 | 4,16 | 21 |
| 21,58 | 4,12 | 12 |
| 22,25 | 3,992 | 15 |
| 22,94 | 3,873 | 32 |
| 23,67 | 3,756 | 9 |
| 24,82 | 3,584 | 7 |
| 25,56 | 3,482 | 37 |
| 26,71 | 3,335 | 9 |
| 27,46 | 3,245 | 4 |
| 28,37 | 3,143 | 8 |
| 30,71 | 2,909 | 3 |
| 29,31 | 3,045 | 4 |
| 29,57 | 3,019 | 4 |
| 31,32 | 2,854 | 10 |
| 32,31 | 2,769 | 4 |
| 33,10 | 2,705 | 5 |
| 33,90 | 2,643 | 1 |
| 34,84 | 2,573 | 2 |
| 35,71 | 2,512 | 1 |
| 36,38 | 2,467 | 1 |
| 36,96 | 2,430 | 1 |
| 37,99 | 2,367 | 2 |
| 39,94 | 2,255 | 5 |

5. Médicament contenant du dimaléate cristallin de 4-[(3-chloro-4-fluorophényl)amino]-6-{[4-(N,N-diméthylamino)-1-oxo-2-butène-1-yl]amino}-7-((*S*)-tétrahydrofuran-3-yloxy)-quinazoline selon la revendication 4, en plus éventuellement d'un ou de plusieurs excipients et/ou diluants inertes.

6. Utilisation du dimaléate cristallin de 4-[(3-chloro-4-fluorophényl)amino]-6-{[4-(N,N-diméthylamino)-1-oxo-2-butène-1-yl]aminol-7-((*S*)-tétrahydrofuran-3-yloxy)-quinazoline selon la revendication 4 pour la préparation d'un médicament, qui est approprié pour le traitement de tumeurs bénignes ou malignes, pour la prévention et le traitement de maladies des voies respiratoires et des poumons ainsi que pour le traitement de maladies du tractus gastro-intestinal et des voies biliaires et de la vésicule biliaire.
